(19) Europäisches Patentamt
European Patent Office
Office européen des brevets

(11) **EP 3 162 425 B1**

(12) **EUROPÄISCHE PATENTSCHRIFT**

(45) Veröffentlichungstag und Bekanntmachung des Hinweises auf die Patenterteilung:
**20.09.2017 Patentblatt 2017/38**

(51) Int Cl.:
***B01D 39/16*** *(2006.01)*

(21) Anmeldenummer: **15192601.1**

(22) Anmeldetag: **02.11.2015**

(54) **FILTERMEDIUM ZUR DEAKTIVIERUNG VON ALLERGENEN**

FILTER MEDIUM FOR DEACTIVATING ALLERGENS

MEDIA FILTRANT DESTINE A LA DESACTIVATION D'ALLERGENES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**03.05.2017 Patentblatt 2017/18**

(73) Patentinhaber: **Carl Freudenberg KG**
**69469 Weinheim (DE)**

(72) Erfinder:
• **SCHACHT, Heiko**
**69469 Weinheim (DE)**
• **HAEFNER, Uwe**
**69469 Weinheim (DE)**
• **STAUDENMAYER, Oliver**
**69469 Weinheim (DE)**

(56) Entgegenhaltungen:
**EP-A1- 2 879 776    US-A1- 2013 183 879**

**EP 3 162 425 B1**

**Beschreibung**

**Technisches Gebiet**

Die Erfindung betrifft ein Filtermedium, insbesondere zur Filtration von Gas-Partikelsystemen, und eine Filteranordnung umfassend das Filtermedium. Die Erfindung betrifft ferner die Verwendung des Filtermediums zur Herstellung von Gebäudezuluft-, Autoinnenraumzuluft- und/oder Raumluftfiltern. Filtermedium und Filteranordnung eignen sich hervorragend zur Deaktivierung von Allergenen.

**Stand der Technik**

Allergene sind Substanzen, die über Vermittlung des Immunsystems Überempfindlichkeitsreaktionen auslösen. Statistiken zeigen, dass die Anzahl der Personen, die von solchen durch Allergene ausgelösten Überempfindlichkeitsreaktionen betroffen ist, von Jahr zu Jahr steigt. Eine Vielzahl von Allergenen, wie beispielsweise Pollen, wird durch die Luft übertragen, was insbesondere für Asthmatiker problematisch ist, die besonders empfindlich auf diese Allergene reagieren. Auch die Anzahl der Asthmatiker steigt von Jahr zu Jahr, was die Situation zusätzlich verschärft.

Es besteht somit ein steigender Bedarf für Filtermaterialien, die Allergene aus der Luft entfernen und vorzugsweise auch neutralisieren können.

In der Praxis werden häufig Polyphenole zur Reduzierung des allergieauslösenden Potenzials der auf Filtermedien abgeschiedenen Stoffe, insbesondere Baumpollen oder Hausstaubmilbenkot, angesehen. So ist aus der EP2879776 (A1) die Verwendung eines Allergendeaktivators, umfassend u.a. Polyphenole aus der Familie der Gerbstoffe, insbesondere organische Gerbsäuren in einem Filtermedium bekannt. Polyphenole haben jedoch den Nachteil, dass sie, beispielsweise auf Filtern, ein vergleichsweise geringes antiallergenes Potenzial über einen längeren Zeitraum aufweisen: Polyphenole als sekundäre Pflanzenstoffe besitzen nämlich überwiegend einen hydrophilen Charakter und können deshalb durch Alterungsvorgänge (große Temperatur- und Feuchteschwankungen) über die Einsatzdauer des Filters abgebaut bzw. ausgewaschen werden

Aus der US 2013/0183879 A1 ist die Verwendung eines Allergendeaktivators, umfassend ein aromatisches Sulfonsäurederivat in einem Filtermedium beschrieben. Als Hilfsmittel werden organische Säuren, insbesondere Zitronensäure, genannt. Aromatische Sulfonsäurederivate wirken in der Regel antibakteriell. Nachteilig an diesen Substanzen ist jedoch, dass sich Resistenzen ausbilden können.

Der Erfindung liegt die Aufgabe zu Grunde ein Filtermedium bereitzustellen, das die oben genannten Probleme zumindest teilweise löst und dazu in der Lage ist, Allergene über einen langen Zeitraum, wie beispielsweise die Einsatzdauer des Filters, effektiv aus der Luft zu entfernen und zu neutralisieren. Darüber hinaus soll das Filtermedium eine bakterizide Wirkung haben und hierdurch für einen langen Zeitraum hygienisch und geruchslos bleiben.

Diese Aufgabe wird gelöst durch ein Filtermedium, insbesondere ein Medium zur Filtration von Gas-Partikelsystemen, umfassend mindestens eine säurefunktionalisierte Lage, die eine Fruchtsäure als erste Säure aufweist, wobei die säurefunktionalisierte Lage als erste Säure eine Fruchtsäure mit einem pks1-Wert von 0 bis 7, vorzugsweise von 1 bis 5, noch bevorzugter von 2 bis 4 und insbesondere von 2,5 bis 3,5 aufweist sowie eine C8- bis C18-Fettsäure als zweite Säure.

Erfindungsgemäß wurde gefunden, dass es die Kombination aus einer Säure mit einem pks1-Wert von 0 bis 7 und C8- bis C18-Fettsäuren ermöglicht, Filtermedien mit einer hohen Kapazität zur Deaktivierung von Allergenen auszustatten. Dies war überraschend, da vermutet wurde, dass Fettsäuren aufgrund ihrer öligen Natur die Aktivität der antiallergenen Säuren blockieren würden. Tatsächlich wurde in praktischen Versuchen eine Reduktion der Deaktivierungsaktivität dieser auf Filtermedien aufgebrachter Säuren gefunden - jedoch in deutlich geringerem Ausmaß als angenommen. Ohne sich erfindungsgemäß auf einen Mechanismus festlegen zu wollen, wird vermutet, dass die beiden Säureklassen dahingehend synergistisch wirken, dass die Fettsäuren als ölige Substanzen die Abscheidung und Fixierung der Allergene auf dem Filtermedium verbessern, wodurch die Blockierung der antiallergenen Säuren zumindest teilweise kompensiert wird.

Darüber hinaus wurde gefunden, dass die Deaktivierung von Allergenen für einen überraschend langen Zeitraum anhalten kann. So hat sich in praktischen (Simulations)versuchen gezeigt, dass durch die Zugabe von C8- bis C18-Fettsäuren eine aufgrund von simulierten Alterungsvorgängen (beispielsweise großen Temperatur- und Feuchteschwankungen) auftretende Reduzierung der Allergen-Deaktivierungsaktivität durch Auswaschung der ersten, antiallergen wirksamen, Säure vermindert werden kann.

Darüber hinaus wurde überraschend gefunden, dass die erfindungsgemäß eingesetzten ersten Säuren auch nach einem Trocknungsvorgang eine hohe Verfügbarkeit im Filtermedium aufweisen. Dies ist ein großer Vorteil verglichen mit Polyphenolen, deren Allergen-Deaktivierungsaktivität bei Trocknungsprozessen der Filtermedienherstellung zum Teil verloren gehen kann.

Als erste Säure können die verschiedensten Fruchtsäuren mit einem pks1-Wert von 0 bis 7, vorzugsweise von 1 bis 5, noch bevorzugter von 2 bis 4 und insbesondere von 2,5 bis 3,5, ausgewählt werden. Dabei kann der pks-Wert mittels Säure-Base-Titration bestimmt werden.

Gemäß einer bevorzugten Ausführungsform der Erfindung ist die erste Säure eine in Wasser lösliche bis sehr leicht lösliche Säure. Hierdurch kann eine hohe Verfügbarkeit der aktiven Komponente sichergestellt werden.

Vorteilhafter Weise wird die erste Säure in wässriger oder alkoholischer Lösung auf die zu funktionalisierende Lage aufgebracht. Dies ist eine besonders einfache Vorgehensweise.

Als besonders geeignet haben sich Äpfelsäure, Fumarsäure, Gluconsäure, Glycolsäure, Mandelsäure, Milchsäure, Oxalsäure, Salicylsäure, α-Hydroxycaprylsäure, Weinsäure, Citronensäure und Gemische hiervon erwiesen. Erfindungsgemäß besonders bevorzugt ist die Verwendung von Citronensäure.

Grundsätzlich ist es denkbar, dass die erste Säure und die zweite Säure die gleichen Verbindungen sind. Erfindungsgemäß bevorzugt sind sie jedoch verschiedene Verbindungen.

Als zweite Säure können erfindungsgemäß die verschiedensten Fettsäuren oder Gemische hiervon eingesetzt werden. Fettsäuren sind aliphatische Monocarbonsäuren mit zumeist unverzweigter Kohlenstoffkette. Als besonders geeignet haben sich erfindungsgemäß Fettsäuren, ausgewählt aus der Gruppe bestehend aus C8- bis C18-Fettsäuren, C8- bis C16-Fettsäuren, C12- bis C14-Fettsäuren, C8-,C10-, C12-Fettsäuren und Gemische hiervon erwiesen. Vorzugsweise weisen die Fettsäuren eine unverzweigte Kohlenstoffkette auf. Die Fettsäuren können gesättigt oder ungesättigt sein oder Gemische von gesättigten und ungesättigten Fettsäuren umfassen. Erfindungsgemäß bevorzugt sind gesättigte Fettsäuren und/oder ungesättigte Fettsäuren mit 1 bis 5 Doppelbindungen.

Als besonders wirksam hat sich die Verwendung von Caprylsäure, Pelargonsäure, Caprinsäure, Undecansäure, Laurinsäure, Tridecansäure, Myristinsäure, Pentadecansäure, Palmitinsäure und/oder Gemischen hiervon als zweite Säure erwiesen.

Ebenso geeignet sind Fettsäurederivate, insbesondere Fettsäuren, die Hydroxy-Gruppen als funktionelle Reste enthalten, sowie Fettsäureester, Fettsäureamide, insbesondere Ölsäureamide und Stearinsäureamide und/oder Gemische hiervon.

Die Moleküle der häufigsten Fettsäuren besitzen 16 oder 18 Kohlenstoffatome. Diese sind deshalb besonders preisgünstig. Zusätzlich haben die Natrium- und Kaliumsalze dieser Fettsäuren den Vorteil als Tensid zu fungieren.

Erfindungsgemäß besonders geeignet sind in Wasser wenig lösliche bis praktisch unlösliche C8- bis C18-Fettsäuren als zweite Säure.

Erfindungsgemäß bevorzugt wird Laurinsäure. Laurinsäure ist eine sehr milde antimikrobielle Substanz und hierdurch in ihrer Anwendung keinen starken Regulatorien unterworfen. Dennoch zeigt Laurinsäure im erfindungsgemäßen Filtermedium eine sehr zufrieden stellende biozide Wirkung.

Erfindungsgemäß besonders bevorzugt ist die Kombination aus Laurinsäure und Citronensäure. In praktischen Versuchen konnte bestätigt werden, dass diese Kombination einem Filtermedium eine herausragende antiallergene und antimikrobielle Wirkung für einen langen Zeitraum, vorzugsweise über die gesamte Einsatzdauer eines Filters verleihen kann. Dazu kommt, dass beide Verbindungen eine gute Umweltverträglichkeit zeigen und bei ihrer Verarbeitung keine außergewöhnlichen Anforderungen an den Arbeitsschutz stellen.

Das Verhältnis zwischen erster Säure und zweiter Säure in der säurefunktionalisierten Lage kann in Abhängigkeit von der erwünschten Performance des Filtermediums eingestellt werden. Als geeignet haben sich insbesondere Verhältnisse im Bereich von 10.000 : 1 bis 1 : 1, vorzugsweise von 1000 : 1 bis 2 : 1, noch bevorzugter 100 : 1 bis 5 : 1 erwiesen.

Auch der Anteil der ersten Säure und zweiten Säure in der säurefunktionalisierten Lage kann in Abhängigkeit von der erwünschten Performance des Filtermediums eingestellt werden. Als geeignet hat sich insbesondere erwiesen, den Anteil der ersten Säure in der säurefunktionalisierten Lage auf 0,1 Gew.% bis 30 Gew.%, vorzugsweise von 2 Gew.% bis 24 Gew.%, noch bevorzugter von 6 Gew.% bis 18 Gew.%, noch bevorzugter von 7 Gew.% bis 15 Gew. und insbesondere von 8 Gew.% bis 12 Gew.%, und/oder den Anteil der zweiten Säure in der säurefunktionalisierten Lage auf weniger als 10 Gew.%, vorzugsweise von 0,01 Gew.% bis 5 Gew.%, noch bevorzugter von 0,02 Gew.% bis 1 Gew.%, noch bevorzugter von 0,04 Gew.% bis 0,6 Gew.% und insbesondere von 0,08 Gew.% bis 0,12 Gew.%, jeweils bezogen auf das Gewicht der säurefunktionalisierten Lage festzulegen. Es wurde gefunden, dass bei einer größeren Konzentration an Fettsäure die antiallergene Aktivität der ersten Säure zu stark reduziert wird.

In praktischen Versuchen wurde ferner gefunden, dass ein erfindungsgemäßes Filtermedium bereits mit einem vergleichsweise geringen Anteil an Fettsäure eine hervorragende Deaktivierung von allergenen Substanzen kombiniert mit einer bioziden Wirkung zeigt.

Das Versehen der zu funktionalisierenden Lage mit der ersten und der zweiten Säure kann auf verschiedene dem Fachmann bekannte Arten und Weisen, wie mittels Imprägnierung und/oder Beschichtung, beispielsweise Pflatschen, Foulardieren, Sprühen und/oder Tauchen erfolgen. So kann die zu funktionalisierende Lage auf einfache Weise mit einer die erste und zweite Säure enthaltenden Lösung und/oder Suspension imprägniert und/oder beschichtet werden. Ebenfalls denkbar ist die Imprägnierung und/oder Beschichtung der Lage mit einem Gemisch aus Bindemittel, beispielsweise einem thermoplastischen Bindemittel, das die erste und zweite Säure enthält.

Als Trägermaterialien für die säurefunktionalisierte Lage können vorzugsweise Vliesstoffe, Gewebe, Gewirke und/oder Papiere eingesetzt werden. Eine erfindungsgemäß besonders bevorzugte Ausführungsform umfasst mithin die Ausgestaltung der säurefunktionalisierten Lage als imprägnierter und/oder beschichteter Vliesstoff, als imprägniertes und/oder beschichtetes Gewebe, Gewirke und/oder Papier. Dabei ist die Verwendung eines Vliesstoffs erfindungsgemäß besonders bevorzugt.

In einer besonders bevorzugten Ausführungsform der Erfindung wird bei der Herstellung des Filtermediums die zu funktionalisierende Lage vor und/oder gleichzeitig mit dem Aufbringen der zweiten Säure mit einer grenzflächenaktiven Substanz als Netzmittel, vorzugsweise einem oder mehreren nichtionischen Tensiden als Netzmittel, noch bevorzugter mit ethoxylierten Sorbitanfettsäureestern (Polysorbaten) behandelt. Besonders bevorzugt sind Polysorbate, die als Lebensmittelzusatzstoff in der Europäischen Union auf Grundlage der VERORDNUNG (EG) Nr. 1333/2008 DES EUROPÄISCHEN PARLAMENTS UND DES RATES vom 16. Dezember 2008 zugelassen sind, wie beispielsweise E 432, E 434, E 435 und E 436. Ebenfalls bevorzugt sind Polysorbate, die in der pharmazeutischen Industrie zur Virusinaktivierung verwendet werden, wie Polysorbat 80 (E433). Hierdurch kann dem Filtermedium eine weitere Funktionalität verliehen werden. Vorteilhaft an der Verwendung von Netzmitteln ist, dass die erste und/oder zweite Säure besonders gut auf der zu funktionalisierenden Lage verankert werden kann. Dies ermöglicht eine gute Immobilisierung und Deaktivierung der Allergene. Im Bezug auf die Verwendung von geruchsintensiven Wirksubstanzen bietet die grenzflächenaktive Substanz den zusätzlichen Vorteil, dass durch die Immobilisierung dieser Substanzen auch die Geruchsfreisetzung verringert werden kann.

Das Filtermedium kann darüber hinaus auch weitere Allergen eliminierende Verbindungen, wie Polyphenole insbesondere Flavonoide, Phenolsäuren, Polyhydroxyphenole, Anthocyane, Procyanidine, Benzoesäure- und Stilbenderivate, vorzugsweise natürlichen Ursprungs, wie beispielsweise die in Granatapfel, Gingko oder Traubenkernmehl befindlichen pflanzlichen Sekundärstoffe und/oder Gemische hiervon enthalten. Dabei liegen diese Verbindungen vorzugsweise in einer Menge von 2% bis 20%, jeweils bezogen auf das Gesamtgewicht des Filtermediums vor.

Das Filtermedium kann auch fungizide Wirkstoffe enthalten. Hierzu kann die säurefunktionalisierte Lage vor und/oder gleichzeitig mit dem Aufbringen der Fettsäure mit einer fungiziden Substanz, vorzugsweise mit Triazolen wie insbesondere 2-Octyl 2H Isothiazol-3-on und/oder Metallen und deren Verbindungen z.B. Zink-Pyrethionen behandelt werden.

Das Filtermedium kann ein- oder mehrlagig vorliegen.

Das erfindungsgemäße Filtermedium eignet sich hervorragend zur Herstellung von Filtern, insbesondere von Gebäudezuluft-, Autoinnenraumzuluft- und/oder Raumluftfiltern.

Ein weiterer Gegenstand der vorliegenden Erfindung ist eine Filteranordnung umfassend ein wie oben beschriebenes Filtermedium. In einer bevorzugten Ausführungsform der Erfindung weist die Filteranordnung einen partikelfilternden Bereich und/oder einen absorbierenden Bereich auf, wobei das Filtermedium von einem oder beiden dieser Bereiche umfasst sein kann.

In einer besonders bevorzugten Ausgestaltung der Erfindung weist die Filteranordnung folgende Bestandteile auf:

A) einen partikelfilternden Bereich, umfassend

- eine Partikelfilterträgerlage, sowie
- eine auf der Partikelfilterträgerlage angeordnete Mikrofaserlage und/oder Membranfilterlage,
- gegebenenfalls eine auf der der Partikelfilterträgerlage abgewandten Seite der Mikrofaserlage und/oder Membranfilterlage angeordnete Decklage; und/oder

B) einen absorbierenden Bereich, umfassend

- eine Adsorptionsschicht, sowie
- eine auf der Adsorptionsschicht angeordnete Adsorptionsträgerlage,

wobei mindestens eine Lage ausgewählt aus Partikelfilterträgerlage, Mikrofaserlage, Membranfilterlage, Decklage, Adsorptionsschicht und Adsorptionsträgerlage aus einem wie oben beschriebenen Filtermedium gebildet ist.

Unter "Partikelfilterträgerlage" ist erfindungsgemäß eine Lage zu verstehen, die als Trägerlage für eine Mikrofaserlage und/oder Membranfilterlage dienen kann.

Unter "Membranfilterlage" ist erfindungsgemäß eine Lage zu verstehen, die eine permeable Membran darstellt.

Unter "Decklage" ist erfindungsgemäß eine Lage zu verstehen, die zur Abdeckung und Schutz der Mikrofaserlage und/oder Membranfilterlage dienen kann.

Unter Adsorptionsschicht ist erfindungsgemäß eine Lage zu verstehen, die ein Adsorptionsmittel aufweist. Dieses ist vorzugsweise ausgewählt aus der Gruppe bestehend aus Aktivkohlepartikeln, Zeolithen, Ionentauschern und Gemischen hiervon. Die Anordung des Adsorptionsmittels in der Adsorptionsschicht erfolgt vorteilhafterweise statistisch regellos als durchströmbare Schüttschicht auf der Adsorptionsträgerlage.

Unter Adsorptionsträgerlage ist erfindungsgemäß eine Lage zu verstehen, die als Trägerlage für die Adsorptionsschicht dienen kann.

Der adsorbierende Bereich der Filteranordnung kann auch aus einer geometrisch bestimmten Anordnung des Adsorptionsmittels beispielsweise als durchströmbarer Wabenkörper definierter Zellgeometrie und/oder Verwendung einer geometrisch definierten Trägerstruktur zur mechanischen Stabilisierung einer Adsorptionsschicht bestehen.

Es ist denkbar, dass die Filteranordnung lediglich den partikelfilternden Bereich oder den absorbierenden Bereich umfasst. Vorteilhafter Weise weist die Filteranordnung jedoch sowohl den partikelfilternden Bereich als auch den absorbierenden Bereich auf, da hierdurch eine besonders effektive Filteranordnung erhalten wird. In diesem Fall werden die beiden Bereiche vorzugsweise so angeordnet, dass die Adsorptionsschicht auf der der Partikelfilterträgerlage abgewandten Seite der Mikrofaserlage, der Membranfilterlage oder der Decklage angeordnet ist. Darüber hinaus ist die Filteranordnung im Einsatz vorzugsweise so angeordnet, dass der partikelfilternde Bereich dem absorbierenden Bereich in Bezug auf die Strömungsrichtung vorgeschaltet ist. Hierdurch können im absorbierenden Bereich vorhandene Wirkstoffe, beispielsweise die erste und zweite Säure, vor der Belegung mit Fremdpartikeln aus der Zuluft geschützt werden.

Erfindungsgemäß ist mindestens eine Lage ausgewählt aus Partikelfilterträgerlage, Mikrofaserlage, Membranfilterlage, Decklage, Adsorptionsschicht und Adsorptionsträgerlage aus einem wie oben beschriebenen Filtermedium gebildet und weist mithin die erfindungsgemäße Kombination aus erster und zweiter Säure auf. Die vorangehenden beschriebenen spezifischen Ausgestaltungen des Filtermediums können dabei auf die jeweils entsprechenden Lagen der Filteranordnung übertragen werden. Grundsätzlich kann lediglich eine einzige Lage oder auch verschiedene Lagen der Filteranordnung die erfindungsgemäße Kombination aus erster und zweiter Säure aufweisen.

Vorteilhaft an dem Einbringen der ersten und zweiten Säure in die Partikelfilterträgerlage ist, dass diese dem Luftstrom üblicherweise als erste Lage der Filteranordnung zugewandt ist und somit allergenhaltige Partikel und Stäube des Luftstroms vor dem Eindringen in die tieferliegenden Schichten der Filteranordung deaktivert werden können.

In einer bevorzugten Ausführungsform der Erfindung ist die erste und zweite Säure in der Decklage enthalten. Vorteilhaft an dieser Ausführungsform ist, dass die in der Filteranordung vorgelagerten Lagen bezüglich ihrer filtertechnischen Eigenschaften nicht beeinflusst werden. Darüberhinaus können auch hier die erste und zweite Säure vor der Belegung mit Fremdpartikeln aus der Zuluft geschützt werden. Diese Anordnung kann noch vorteilhafter sein, wenn die erste und zweite Säure weder in der Partikelfilterträgerlage noch in der Mikrofaserlage oder der Membranfilterlage vorliegt.

Vorteilhaft an dem Einbringen der ersten und zweiten Säure in die Adsorptionsschicht ist, dass Adsorptionsschichten generell hohe spezifische Oberflächen bereitstellen (bei Verwendung von Aktivkohle ca. 1000 m$^2$/g) und daher für die Allergen-Deaktivierung eine große reaktive Oberfläche zur Verfügung steht. Darüberhinaus können auch hier die erste und zweite Säure vor der Belegung mit Fremdpartikeln aus der Zuluft durch den partikelfilternden Bereich bzw. durch die Adsorptionsträgerlage geschützt werden.

Vorteilhaft an dem Einbringen der ersten und zweiten Säure in die Adsorptionsträgerlage ist, dass die in der Filteranordung vorgelagerten Lagen bezüglich ihrer filtertechnischen Eigenschaften durch das Einbringen der ersten und zweiten Säure in die Adsorptionsträgerlage nicht beeinflusst werden. Darüberhinaus können die erste und zweite Säure vor der Belegung mit Fremdpartikeln aus der Zuluft durch den partikelfilternden Bereich geschützt werden.

In einer erfindungsgemäß besonders bevorzugten Ausführungsform weist die Filteranordnung in Bezug auf die Strömungsrichtung folgenden Aufbau auf: Partikelfilterträgerlage, Mikrofaserlage, Adsorptionsschicht und Adsorptionsträgerlage. Dabei ist die Partikelfilterträgerlage im Einsatz vorteilhafter Weise anströmseitig angeordnet.

Wie bereits oben erläutert, können die Trägermaterialien für Partikelfilterträgerlage, Mikrofaserlage, Membranfilterlage, Decklage und Adsorptionsträgerlage vorteilhafter Weise Vliesstoffe, Gewebe, Gewirke und/oder Papiere sein.

Als geeignet hat sich ferner erwiesen, den Anteil der ersten Säure in der Filteranordnung auf 0,003 Gew.% bis 30 Gew.%, vorzugsweise von 0,1 Gew.% bis 24 Gew.% noch bevorzugter von 0,2 Gew.% bis 18 Gew.% noch bevorzugter von 0,25 Gew.% bis 15 Gew.% und insbesondere von 0,3 Gew.% bis 12 Gew.% und/oder den Anteil der zweiten Säure in der Filteranordnung auf 0,0001 Gew.% bis 10 Gew.% noch bevorzugter von 0,0003 Gew.% bis 5 Gew.% noch bevorzugter von 0,0006 Gew.% bis 1 Gew.% noch bevorzugter von 0,001 Gew.% bis 0,6 Gew.% und insbesondere von 0,003 Gew.% bis 0,12 Gew.%, jeweils bezogen auf das Gesamtgewicht der Filteranordnung festzulegen.

In bevorzugter Ausgestaltung der Erfindung weist die Adsorptionsträgerlage und/oder die Partikelfilterträgerlage einen Vliesstoff auf, vorzugsweise ausgewählt aus Spinnvliesstoffen, mit einem mittleren Faserdurchmesser im Bereich von 20 bis 70 μm, vorzugsweise von 20 bis 50 μm, insbesondere von 20 bis 50 μm und/oder Stapelfaservliesstoffen mit einem mittleren Faserdurchmesser von 5 bis 60 μm, vorzugsweise von 10 bis 50 μm, insbesondere von 10 bis 35 μm und/oder einer mittleren Faserlänge von 10 bis 100 mm, vorzugsweise von 30 bis 80 mm. Weiter vorteilhaft weist die Mikrofaserlage und/oder Membranfilterlage einen Vliesstoff, vorzugsweise ausgewählt aus Melt-blownfaservliesstoffen mit einem mittleren Faserdurchmesser von 1 μm bis 10 um auf. Weiter vorteilhaft weist die Decklage einen Vliesstoff, vorzugsweise ausgewählt aus Spinnvliesstoffen, mit einem mittleren Faserdurchmesser im Bereich von 20

bis 60 $\mu$m und/oder Stapelfaservliesstoffen mit einem mittleren Faserdurchmesser von 10 bis 50 $\mu$m auf.

Eine erfindungsgemäß besonders bevorzugte Ausführungsform umfasst die Ausgestaltung der Adsorptionsträgerlage, der Partikelfilterträgerlage, der Mikrofaserlage, der Membranfilterlage und/oder der Decklage als mit der ersten und zweiten Säure imprägnierten und/oder beschichteten Vliesstoff, wie oben beschrieben.

Das erfindungsgemäße Filtermedium und die Filteranordnung eignen sich hervorragend zur Filterung von Gebäudezuluft, Autoinnenraum-Zuluft und/oder Raumluft und dabei insbesondere zur Filterung von Autoinnenraum-Zuluft.

**Kurzbeschreibung der Figuren**

In der Zeichnung zeigt:

Fig.1 Eine erfindungsgemäße Filteranordnung mit einem absorbierenden Bereich 1 und einem partikelfilternden Bereich 2. Der partikelfilternde Bereich 2 umfasst hier eine mit Laurinsäure und Citronensäure imprägnierte Partikelfilterträgerlage 1a.

Fig.2 Eine weitere erfindungsgemäße Filteranordnung mit einem absorbierenden Bereich 1 und einem partikelfilternden Bereich 2, Der partikelfilternde Bereich 2 umfasst hier eine mit Laurinsäure und Citronensäure imprägnierte Decklage 1c.

**Figurenbeschreibung**

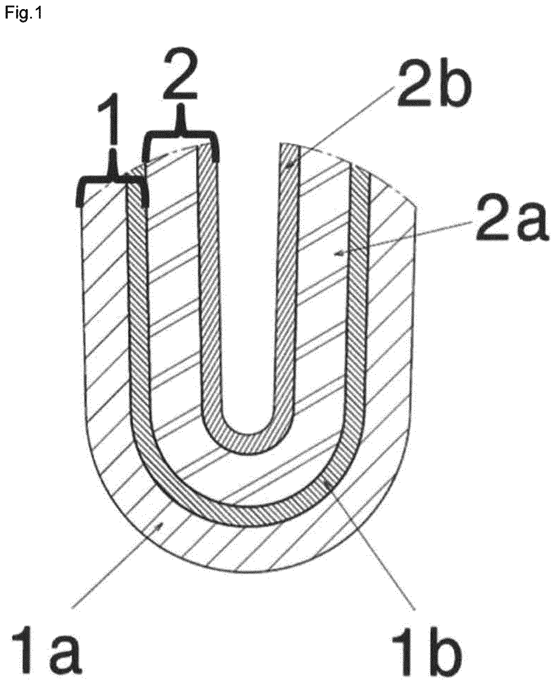

Figur 1 zeigt eine erfindungsgemäße Filteranordnung umfassend einen partikelfilternden Bereich 1, der

A) eine Partikelfilterträgerlage 1a, sowie

- eine auf der Partikelfilterträgerlage 1a angeordnete Mikrofaserlage 1b, sowie

B) einen absorbierenden Bereich 2 aufweist, umfassend

- eine auf der der Partikelfilterträgerlage 1a abgewandten Seite der Mikrofaserlage 1b angeordnete Adsorptionsschicht 2a, sowie
- eine auf der der Mikrofaserlage 1b abgewandten Seite der Adsorptionsschicht 2a angeordnete Adsorptionsträgerlage 2b.

In dieser Ausgestaltung ist die Partikelfilterträgerlage 1a als mit Laurinsäure und Citronensäure imprägnierter Vliesstoff ausgebildet.

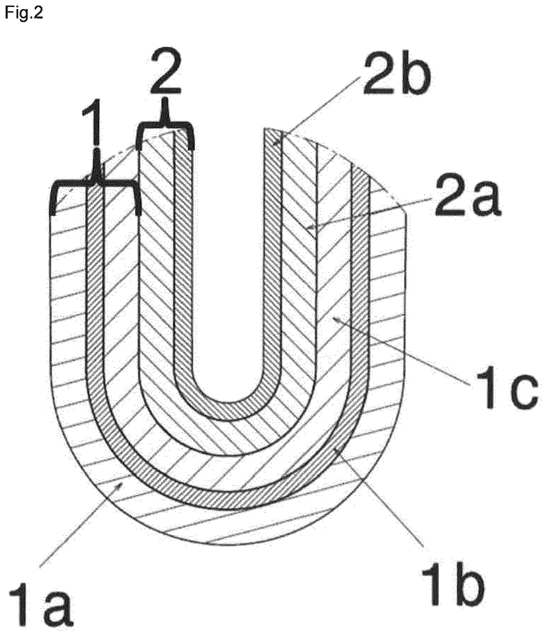

Figur 2 zeigt eine erfindungsgemäße Filteranordnung umfassend einen partikelfilternden Bereich 1, der

A) eine Partikelfilterträgerlage 1a, sowie

- eine auf der Partikelfilterträgerlage 1a angeordnete Mikrofaserlage 1b,
- und eine auf der der Partikelfilterträgerlage 1a abgewandten Seite der Mikrofaserlage 1b angeordnete Decklage (1c); sowie

B) einen absorbierenden Bereich 2 aufweist, umfassend

- eine auf der der Mikrofaserlage 1b abgewandten Seite der Decklage (1c) angeordnete Adsorptionsschicht 2a, sowie
- eine auf der der Decklage (1c) abgewandten Seite der Adsorptionsschicht 2a angeordnete Adsorptionsträgerlage 2b.

In dieser Ausgestaltung ist die Adsorptionsträgerlage 2b als mit Laurinsäure und Citronensäure imprägnierter Vliesstoff ausgebildet.

**Messmethoden:**

**Messung der pks-Werte**

Die pks-Werte in der vorliegenden Erfindung können durch Auswertung der mittels Säure - Basen Titration erhaltenen Titrationskurven bestimmt werden (Daniel C. Harris, Lehrbuch der quantitativen Analyse, Springer Verlag 2014).

**Einstufung der Wasserlöslichkeit der Säuren**

Die verbale Einstufung der Wasserlöslichkeit in der vorliegenden Erfindung erfolgt in Anlehnung an das Europäische Arzneibuch (8. Ausgabe, 3. Nachtrag, Amtliche deutsche Ausgabe).

| Verbale Einstufung der Wasserlöslichkeit | Wasserlöslichkeit der Substanz in g / l |
|---|---|
| sehr leicht löslich | > 1000 |
| leicht löslich | 100 bis 1000 |
| löslich | 33 bis 100 |
| wenig löslich | 10 bis 33 |
| schwer löslich | 1 bis 10 |
| sehr schwer löslich | 0,1 bis 1 |
| praktisch unlöslich | < 0,1 |

Die Messung der Löslichkeit erfolgt dabei gemäß VERORDNUNG (EG) Nr. 440/2008 DER KOMMISSION vom 30. Mai 2008 zur Festlegung von Prüfmethoden gemäß der Verordnung (EG) Nr. 1907/2006 des Europäischen Parlaments und des Rates zur Registrierung, Bewertung, Zulassung und Beschränkung chemischer Stoffe (REACH)

**TEIL A: METHODEN ZUR BESTIMMUNG DER PHYSIKALISCH-CHEMISCHEN EIGENSCHAFTEN**

A.6. WASSERLÖSLICHKEIT

**Vorversuch**

Etwa 0,1 g der Probe (feste Substanzen müssen pulverisiert sein) werden in einem mit Glasstopfen verschließbaren 10-ml-Messzylinder gegeben. Gemäß der Tabelle wird portionsweise destilliertes Wasser von Raumtemperatur zugesetzt.

| ml Wasser in 0,1g lösliche Substanz | 0,1 | 0,5 | 1 | 2 | 10 | 100 | >100 |
|---|---|---|---|---|---|---|---|
| ungefähre Löslichkeit in Wasser (g/l) | >100 0 | 1000-2000 | 200-100 | 100-50 | 50-10 | 10-1 | <1 |

Nach jedem Zusatz der in der Tabelle angegebenen Wassermenge wird die Mischung 10 Minuten kräftiggeschüttelt und mit bloßem Auge auf ungelöste Teilchen untersucht. Wenn nach Zusatz von 10 ml Wasser die Probe oder Teile von ihr ungelöst bleiben, ist der Versuch in einem 100-ml-Messzylinder mit größeren Mengen Wasser zu wiederholen. Bei geringer Wasserlöslichkeit kann die zur Auflösung einer Substanz erforderliche Zeit erheblich länger sein (bis zu 24 Stunden). Die ungefähre Wasserlöslichkeit ist in der Tabelle angegeben, und zwar unter dem Volumen des zur vollständigen Auflösung der Probe notwendigen Wassers. Ist die Substanz noch immer nicht vollständig gelöst, sollte der Versuch länger als 24 Stunden (maximal 96 Stunden) durchgeführt oder es sollte weiter verdünnt werden, um festzustellen, ob entweder die Säulen-Elutions- oder die Kolben- Methode zu benutzen ist.

Die (beiden nachstehend beschriebenen) Prüfmethoden decken den gesamten Bereich der Wasserlöslichkeiten ab, eignen sich jedoch nicht für flüchtige Stoffe:

- eine Prüfmethode für im Wesentlichen reine Substanzen geringer Wasserlöslichkeit ($< 10^{-2}$ g/l), die in Wasser stabil sind; diese Methode wird als "Säulen-Elutions-Methode" bezeichnet.

- Die andere Prüfmethode eignet sich für im Wesentlichen reine Stoffe höherer Wasserlöslichkeit (> $10^{-2}$ g/l), die in Wasser stabil sind; diese Methode wird als "Kolben-Methode" bezeichnet.

Beschreibung der Methode:

### Prüfbedingungen

Die Prüfung wird vorzugsweise bei 20°C $\pm$ 0,5 oC durchgeführt. Wenn eine Temperaturabhängigkeit der Wasserlöslichkeit > 3 % / °C vorzuliegen scheint, wird bei zwei weiteren Temperaturen, die mindestens 10 °C unter und über der ursprünglich gewählten Temperatur liegen, ebenfalls gemessen. In diesem Fall sollte die Temperaturkonstanz bei $\pm$ 0,1°C liegen. Die gewählte Temperatur soll in den wichtigen Teilen der Apparatur konstant gehalten werden.

### Beispiele

Im Folgenden wird die Erfindung anhand mehrerer, nicht beschränkender, Beispiele näher erläutert:

In den Beispielen wird ELISA (Enzyme-Linked ImmunoSorbent Assay) als ein Verfahren zur Messung der Deaktivierungsfähigkeiten verschiedener Wirkstoff-Zusammensetzungen im Hinblick auf bestimmte Allergene verwendet.

Das ELISA-Verfahren kann Allergenkonzentrationen durch Bewertung von Farbänderungen aufgrund einer Antigen-Antikörper-Reaktion messen.

Jedem Beispiel liegen Versuche unter Verwendung der erfindungsmäßig genannten Wirkstoffe in unterschiedlichen Gewichtsanteilen für spezifische Allergene zugrunde.

### 1. Probenvorbereitung

Ein Trägervlies ausPolyester-Spinnvliesstoff (Flächengewicht 125 g/m$^2$) wird mit einem Gemisch aus Laurinsäure und Citronensäure allergen-deaktivierend ausgerüstet. Die allergen-deaktivierende Dotierung des Trägervlieses erfolgt hierbei durch Aufbringen einer Mischung der Wirkstoffe in wässriger Lösung auf das Trägervlies und nachfolgende Trocknung des nunmehr ausgerüsteten Vliesstoffs, um dadurch eine Probe für eine Analyse zu erhalten.

Die Größe der im Test verwendeten Probe ist 10 mm $\times$ 10 mm.

### 1.1 Herstellung der antigenhaltigen Ausgangslösung

Verwendete Allergenquellen und zu prüfende Antigene:

a) Allergen Hausstaubmilben: Antigen Der p 1 aus Exkrementen
b) Allergen Birkenpollen: Antigen Bet v 1 aus Pollen

Es werden Allergenquellen der Fa. Allergon AB, Ängelholm, Schweden verwendet. Zur Vorbereitung einer Testallergenlösung definierter Ausgangskonzentration wird jede Allergenquelle nach Vorgabe des ELISA-Kit Herstellers Indoor Biotechnologies Inc., Charlottesville, USA in PBS-Pufferlösung aufgelöst, um dadurch eine Testallergenlösung mit definiertem Ausgangs-Antigen-Gehalt herzustellen. Andere Reagenzien werden nach den Angaben des ELISA-Kit Herstellerangabe verwendet.

### 1.2 Prüfung des Filtermediums auf dessen allergen-deaktivierende Wirksamkeit

Jede in 10 mm $\times$ 10 mm große Stücke geschnittene Probe wird in 300 $\mu$l der Allergenlösung bekannter Ausgangs-Antigen-Konzentration bei 25 ° C für 1 Stunde eingeweicht.

Ein ELISA-Testkit wird für jedes Antigen verwendet.

Nach einstündigem Einweichen werden 100 $\mu$l der Lösung (Überstand) in eine mit Antikörpern beschichteten 96-Well-Mikroplatte pipettiert.

Die Absorption der Mikroplatte wird entsprechend der Vorgabe des ELISA-Kit-Herstellers unter Verwendung eines Mikroplattenlesegeräts bei einer Wellenlänge von 405nm ermittelt und somit die Allergen-Konzentration (Antigen-Gehalt) jeder Probe bestimmt.

### 1.3 Messung der Testergebnisse

Um die Allergen-deaktivierende Wirksamkeit zu bestimmen, wird jede Antigen-Konzentration der Proben-Reaktionslösung entsprechend der Vorgabe des ELISA-Kit-Herstellers unter Verwendung eines Mikroplatten-Lesegeräts bei einer Wellenlänge von 405nm ermittelt.

$$\text{Wirkungsgrad (\%)} = \frac{\text{Ausgangskonzentration} - \text{gemessene Allergenkonzentration der Probe}}{\text{Ausgangskonzentration}}$$

Ausgangskonzentration und gemessene Allergenkonzentration der Probe entsprechen hierbei einer antigen-spezifischen Konzentration in ng/ml.

## 2. Prüfung der immunologischen Wirkung von Fruchtsäuren in Abhängigkeit von Art und Konzentration

Es wurde der Wirkungsgrad der Allergeneliminierung (Birkenallergen; Antigen Bet v1) verschiedener Fruchtsäuren untersucht. Die Ausgangskonzentration entspricht dabei der Zugabe von 303 ng/ml Antigen Bet v1.

Zunächst wurde ein Trägervlies aus Polyester-Spinnvliesstoff (Flächengewicht 125 g/m$^2$) mit Citronensäure bzw. Äpfelsäure unterschiedlicher Konzentrationen allergen-deaktivierend ausgerüstet. Im Ergebnis erhielt man als Material 1 ein mit 10 Gew.% Citronensäure ausgerüstetes Filtermedium, als Material 3 ein mit 1 Gew.% Citronensäure ausgerüstetes Filtermedium, als Material 4 ein mit 10 Gew.% Äpfelsäure ausgerüstetes Filtermedium und als Material 5 ein mit 1 Gew.% Äpfelsäure ausgerüstetes Filtermedium.

Das Ergebnis ist in nachfolgender Tabelle dargestellt:

**Tab.1**

| Material | Antigenzugabe | Wirkungsgrad |
|---|---|---|
| Material 1 | 303 ng/ml Bet v1 | 81% |
| Material 3 | 303 ng/ml Bet v1 | 10% |
| Material 4 | 303 ng/ml Bet v1 | 83% |
| Material 5 | 303 ng/ml Bet v1 | 15% |

Es zeigt sich, dass Citronensäure und Äpfelsäure eine hohe Allergen eliminierende Wirkung haben, die mit steigender Konzentration zunimmt. Überraschend ist, dass die Aktivität der Säuren auch im getrockneten Zustand vorliegt, was die hohe Stabilität der Säuren belegt.

## 3. Elisa-Prüfung: Fruchtsäuren in Kombination mit Fettsäuren und weiteren Allergen eliminierenden Substanzen

Es wurde der Wirkungsgrad der Allergeneliminierung (Milbenkotallergen; Antigen Der p1) verschiedener Fruchtsäuren in Kombination mit Fettsäuren untersucht. Die Ausgangskonzentration entspricht dabei der Zugabe von 232 ng/ml Antigen Der p1.

Zunächst wurde ein Trägervlies aus Polyester-Spinnvliesstoff (Flächengewicht 125 g/m$^2$) mit Citronensäure, Laurinsäure, Detergenz Tween 80 und Traubenkernmehl unterschiedlicher Konzentrationen allergen-deaktivierend ausgerüstet. Im Ergebnis erhielt man als Material 6 ein mit 5 Gew.% Citronensäure ausgerüstetes Filtermedium, als Material 7 ein mit 5 Gew.% Citronensäure und 5 Gew.% Laurinsäure ausgerüstetes Filtermedium, als Material 8 ein mit 5 Gew.% Citronensäure, 5 Gew.% Laurinsäure und 0,5% Tween 80 ausgerüstetes Filtermedium und als Material 9 ein mit 5 Gew.% Citronensäure, 5 Gew.% Laurinsäure, 0,5 Gew.% Tween 80 und 10 Gew.% Traubenkernmehl ausgerüstetes Filtermedium.

Das Ergebnis ist in Tabelle 2 dargestellt.

**Tab. 2**

| Material | Antigenzugabe | Wirkungsgrad |
|---|---|---|
| Material 6 | 232 ng/ml Der p1 | 68% |
| Material 7 | 232 ng/ml Der p1 | 42% |
| Material 8 | 232 ng/ml Der p1 | 11% |
| Material 9 | 232 ng/ml Der p1 | 21% |

Die Ergebnisse zeigen, dass durch die Zugabe der Fettsäure die Verfügbarkeit der Fruchtsäuren zwar reduziert wird, jedoch noch ein zufrieden stellender Wirkungsgrad erhalten bleibt. Auch durch die Zugabe relativ hoher Konzentrationen an Detergenzes Tween 80 findet, wie erwartet, eine Reduzierung der Verfügbarkeit der Fruchtsäuren statt, die jedoch nicht zur völligen In-Aktivierung führt.

Durch die Zugabe von Polyphenolen, beispielsweise Mischungen aus Flavonoiden, Phenolsäuren, Polyhydroxyphenole, Anthocyanen, Procyanidinen sowie Benzoesäure- und Stilbenderivaten beispielsweise natürlichen Ursprungs, wie Traubenkernmehl, kann die Allergen eliminierende Wirkung verstärkt werden.

**4. Elisa-Prüfung: Optimierung der Wirkstoffkombinationen**

Es wurde der Wirkungsgrad der Allergeneliminierung (Milbenkotallergen; Antigen Der p1) optimierter Wirkstoffkombinationen untersucht. Die Ausgangskonzentration entspricht dabei Zugabe von 148 ng/ml Antigen Der p1.

Wie in Figur 2 dargestellt, können besonders gute immunologische Wirkungen der Citronensäure erhalten werden, wenn ihre Konzentration mehr als 5 Gew.%, hier 10 Gew.% beträgt. Ebenfalls von Vorteil ist es, wenn die Laurinsäure in einer Menge von weniger als 10 Gew.%, vorzugsweise von 0,01 Gew.% bis 1 Gew.%, jeweils bezogen auf das Gewicht der säurefunktionalisierte Lage vorliegt.

Zunächst wurde ein Trägervlies aus Polyester-Spinnvliesstoff (Flächengewicht 125 g/m$^2$) mit Citronensäure, Laurinsäure und Detergenz Tween 80 in optimierten Konzentrationensverhältnissen allergen-deaktivierend ausgerüstet. Im Ergebnis erhielt man als Material 10 ein mit 10 Gew.% Citronensäure und 1 Gew.% Laurinsäure ausgerüstetes Filtermedium, als Material 11 ein mit 10 Gew.% Citronensäure, 1 Gew.% Laurinsäure und 0,5% Tween 80 ausgerüstetes Filtermedium und als Material 12 ein mit 10 Gew.% Citronensäure, 0,1 Gew.% Laurinsäure und 0,5 Gew.% Tween 80 ausgerüstetes Filtermedium.

**Tab.3**

| Material | Antigenzugabe | Wirkungsgrad |
|---|---|---|
| Material 10 | 148 ng/ml Der p1 | 91% |
| Material 11 | 148 ng/ml Der p1 | 90% |
| Material 12 | 148 ng/ml Der p1 | 91% |

**5. ELISA-Prüfung (Vergleich mit Vergleichswirkstoff Kaliumcarbonat)**

Es wurde der Wirkungsgrad der Allergeneliminierung (Milbenkotallergen;Antigen Der p1) von Kaliumcarbonat als Vertreter basischer Antiallergene getestet. Die Ausgangskonzentration entspricht dabei der Zugabe von 140 ng/ml Antigen Der p1.

Zunächst wurde ein Trägervlies aus Polyester-Spinnvliesstoff (Flächengewicht 125 g/m$^2$) mit Kaliumcarbonat, Laurinsäure und Detergenz Tween 80 in definierten Konzentrationensverhältnissen allergen-deaktivierend ausgerüstet.

Im Ergebnis erhielt man als Material 13 ein mit 5 Gew.% Kaliumcarbonat ausgerüstetes Filtermedium, als Material 14 ein mit 5 Gew.% Kaliumcarbonat, 0,1 Gew.% Laurinsäure und 0,5% Tween 80 ausgerüstetes Filtermedium, als Material 15 ein mit 10 Gew.% Kaliumcarbonat ausgerüstetes Filtermedium und als Material 16 ein mit 10 Gew.% Kaliumcarbonat, 0,1 Gew.% Nie Laurinsäure und 0,5 Gew.% Tween 80 ausgerüstetes Filtermedium.

**Tab.4**

| Material | Antigenzugabe | Wirkungsgrad |
|---|---|---|
| Material 13 | 140 ng/ml Der p1 | 15% |
| Material 14 | 140 ng/ml Der p1 | 20% |
| Material 15 | 140 ng/ml Der p1 | 24% |
| Material 16 | 140 ng/ml Der p1 | 36% |

Wie in Tabelle 4 dargelegt, ist die immunologische Wirkung von Kaliumcarbonat deutlich geringer als die der erfindungsgemäß eingesetzten Säuren.

**6. Mikrobielle Prüfung**

Die mikrobielle Prüfung eines erfindungsgemäßen Filtermediums wird entsprechend des Übertragungsverfahrens gemäß Norm International Standards Organization (ISO) 20743:2013 (Textiles - Determination of antibacterial activity of textile products) wie folgt durchgeführt: Eine Suspension aus definierten Bakterienstämmen mit bekannter Zellkonzentration wird auf eine Agarplatte einer Petrischale ausgestrichen. Das erfindungsgemäße Filtermedium wird auf die Petrischale gelegt. Zur Übertragung der Bakterien auf das Filtermedium wird selbiges mit einem Gewicht von 200 g für einen Zeitraum von 1 min beschwert. Anschließend wird das derart mit Bakterien beaufschlagte Filtermedium für 5 Minuten bei 37 °C angebrütet, um die Anfangs-Bakterienkonzentration auf dem Filtermedium in koloniebildenden Einheiten zu ermitteln. Dieser Ansatz wurde als Nullpunktwert herangezogen. Die restlichen Proben des Filtermediums wurden anschließend bei 37 °C über einen definierten Zeitraum (18- 24 h) bebrütet, um die mikrobielle Wirksamkeit des Filtermediums zu bestimmen:

Eine antimikrobielle Wirkung des Filtermediums liegt vor, wenn die Bakterienkonzentration auf dem Filtermedium am Ende der Bebrütungsdauer geringer ist als die Bakterienkonzentration am wirkstofffreien Vergleichsmuster (jeweils gemessen in koloniebildenden Einheiten KBE/ml).

Die Berechnung der Wirksamkeit erfolgt über den Reduktionsfaktor und wurde nach Norm International Standards Organization (ISO) 22196:2011 (Measurement of antibacterial activity on plastics and other non-porous surfaces) durchgeführt, da die behandelten Proben so stark antimikrobiell wirkten, dass auch schon nach wenigen Minuten Inkubationszeit eine fast vollständige Abtötung erreicht wurde. Diese Proben konnten daher sinnvollerweise nicht als Nullproben laut ISO 20743 herangezogen werden. Da oftmals in der Praxis keine geeigneten unbehandelten, textilen Vergleichsmuster zur Verfügung stehen ist in diesem Fall eine Berechnung laut Textiliennorm ISO 20743 nicht zweckmäßig, Die Berechnung erfolgte daher wie in ISO 22196 durch Vergleich mit einem unbehandelten Muster bei gleicher Inkubationszeit, dabei berechnet sich der Reduktionsfaktor nach der folgenden Formel:

$$R = [\log(B/A) - \log(C/A)] = [\log(B/C)]$$

R: Wert der antimikrobiellen Aktivität
A: Lebendkeimzahlen an den Teststücken direkt nach dem Beimpfen
B: Lebendkeimzahlen am wirkstofffreien Nullmuster
C: Lebendkeimzahlen am wirkstoffhaltigen Teststück

Im Fall einer vollständigen Abtötung der Bakterien auf dem Prüfmuster, kann der Reduktionsfaktor nicht nach oben angeführter Formel berechnet werden (Unbestimmtheit wegen Division durch 0). Laut Norm 22196 ergibt sich aber dieses Problem nicht, da bei Abarbeitung der Methode in diesen Fall eine Nachweisgrenze von 40 Bakterien gegeben ist (da ein 0,5 ml Aliquot der 20 ml Abspüllösung inkubiert wird und nicht das gesamte Volumen), und in diesem Fall statt 0 die Nachweisgrenze von 40 in die oben genannte Formel eingesetzt wird.

Der Reduktionsfaktor ist ein Maß dafür, wie viele der in der ursprünglichen Bakteriensuspension enthaltenen Mikroorganismen, die im ersten Schritt der Untersuchung direkt auf die ausgerüsteten Materialmuster aufgebracht werden, durch den Kontakt mit dem bioziden Material im Vergleich zum Nullmuster absterben. Da die Bakterienkonzentrationen in Form von Zehnerpotenzen angegeben werden, arbeitet man bei der Berechnung mit den zugehörigen dekadischen Logarithmen.

Ein Reduktionsfaktor von 2 bedeutet daher, dass die Konzentration der Bakteriensuspension nach dem Kontakt mit der beschichteten Probe im Vergleich zur Nullprobe um 2 Zehnerpotenzen geringer ist. Je größer der Reduktionsfaktor ist umso mehr Bakterien sterben während des Kontaktes mit der ausgerüsteten Oberfläche und umso höher ist daher die Wirksamkeit der Beschichtung/Probe/etc.

Die Anzahl der auf das Filtermedium übertragenen Bakterien wird hierbei über das Koch'sche Plattengussverfahren quantifiziert.

Die Messungen erfolgte in Anlehnung an die Norm ISO 20743. Diese Norm ist anwendbar auf alle Textilerzeugnisse und Materialien für Kleidung, Bettwäsche, Heimtextilien und verschiedene Waren, unabhängig von der Art der verwendeten antibakteriellen Wirkstoffe. Abweichend von dieser Norm wurden die Untersuchungen nicht mit dem gramnegativer Referenzkeim Klebsiella pneumoniae, sondern mit dem ebenfalls gramnegativen Keim Escherichia coli durchgeführt:

Die Spezies Escherichia coli ist normalerweise nicht krankheitsauslösend, jedoch existieren auch zahlreiche verschiedene pathogene Stämme, die dabei zu den häufigsten Verursachern von menschlichen Infektionskrankheiten gehören. Ferner wird in der japanischen Norm JIS L1902 - Testing for antibacterial activity and efficacy on textile products -, auf deren Grundlage die Norm ISO 20743 verabschiedet wurde der Keim Escherichia coli als zu heranziehender Referenzkeim benannt. Als Ursache, dass nicht bei allen Textil-Prüfnormen zur antibakteriellen Aktivität Escherichia coli als Referenzkeim herangezogen wird kann seine erhöhte Empfindlichkeit gegenüber Austrocknung angesehen werden, was sich bei den Prüfungen störend auswirken kann. Da im vorliegenden Fall bei rel. Luftfeuchten von 90% bebrütet wurde und sich die Keimzahl der unausgerüsteten Materialprobe über die Bebrütungsdauer erhöht hat (sog. Blindwert) kann dies ausgeschlossen werden.

### 6.1 Mikrobielle Prüfung mit Staphylococcus aureus DSM 799 (grampositiv)

Zunächst wurde ein Trägervlies aus Polyester-Spinnvliesstoff (Flächengewicht 125 g/m$^2$) mit Citronensäure, Laurinsäure und Detergenz Tween 80 in optimierten Konzentrationsverhältnissen allergen-deaktivierend ausgerüstet. Im Ergebnis erhielt man als **Material 11** ein mit 10 Gew.% Citronensäure, 1 Gew.% Laurinsäure und 0,5% Tween 80 ausgerüstetes Filtermedium und als **Material 12** ein mit 10 Gew.% Citronensäure, 0,1 Gew.% Laurinsäure und 0,5 Gew.% Tween 80 ausgerüstetes Filtermedium. Zum Vergleich wurden ebenfalls die Bakterienzahlen für das unausgerüstete Filtermedium aus Polyester-Spinnvliesstoff (Flächengewicht 125 g/m$^2$) ermittelt (**Material 17**).

In der folgenden Tabelle ist die biozide Wirkung verschiedener Filtermedien auf das Bakterium Staphylococcus aureus DSM 799 (grampositiv) nach einer Dauer der Bebrütung von 18h vergleichend dargestellt.

Tab.5

| Material | Reduktionsfaktor im Vergleich zum unbehandelten Material (nach 5 min Einwirkzeit) | Reduktionsfaktor im Vergleich zum unbehandelten Material (nach 18h Bebrütung) |
|---|---|---|
| 11 | 3,7 | 6,7 |
| 12 | 3,3 | 6,7 |
| 17 | - (unbehandeltes Material) | - (unbehandeltes Material) |

Aus Tabelle 5 ergibt sich, dass auch bei geringen Anteilen an Laurinsäure von 0,1 % bereits eine biozide Wirkung erzielt werden kann. Diese Wirkung steigt erwartungsgemäß mit zunehmender Menge an Laurinsäure und/oder bei Verwendung von Tween 80. Entsprechend Norm ISO 20743:2013 liegt für die Materialien 11 und Material 12 eine antimikrobielle Wirkung des Filtermediums vor, da der dekadisch logarithmische Reduktionsfaktor >2 ist.

### 6.2 Mikrobielle Prüfung mit Escherichia coli DSM 787 (gramnegativ)

Zunächst wurde ein Trägervlies aus Polyester-Spinnvliesstoff (Flächengewicht 125 g/m$^2$) mit Citronensäure, Laurinsäure und Detergenz Tween 80 in optimierten Konzentrationsverhältnissen allergen-deaktivierend ausgerüstet. Im Ergebnis erhielt man als **Material 11** ein mit 10 Gew.% Citronensäure, 1 Gew.% Laurinsäure und 0,5% Tween 80 ausgerüstetes Filtermedium und als **Material 12** ein mit 10 Gew.% Citronensäure, 0,1 Gew.% Laurinsäure und 0,5 Gew.% Tween 80 ausgerüstetes Filtermedium. Zum Vergleich wurden ebenfalls die Bakterienzahlen für das unausgerüstete Filtermedium aus Polyester-Spinnvliesstoff (Flächengewicht 125 g/m$^2$) ermittelt (**Material 17**).

In der folgenden Tabelle ist die biozide Wirkung verschiedener Filtermedien auf das Bakterium Escherichia coli DSM 787 (gramnegativ) nach einer Dauer der Bebrütung von 22h vergleichend dargestellt.

Tab.6

| Material | Reduktionsfaktor im Vergleich zum unbehandelten Material (nach 5 min Einwirkzeit) | Reduktionsfaktor im Vergleich zum unbehandelten Material (nach 22h Bebrütung) |
|---|---|---|
| 11 | 3,7 | 4,3 |
| 12 | 3,7 | 4,3 |
| 17 | - (unbehandeltes Material) | - (unbehandeltes Material) |

Aus Tabelle 6 ergibt sich, dass auch bei geringen Anteilen an Laurinsäure von 0,1 % bereits eine biozide Wirkung erzielt werden kann. Entsprechend Norm ISO 20743:2013 liegt für die Materialien 11 und Material 12 eine antimikrobielle Wirkung des Filtermediums vor, da der dekadische logarithmische Reduktionsfaktor >2 ist.

**6.3 Nachstellungen der Verfügbarkeit der ersten Säure in Filtermedien über die Einsatzdauer des Filters**

Als Messgröße für die Verfügbarkeit der ersten Säure in Filtermedien über die Einsatzdauer des Filters eignet sich die Bestimmung des pH-Werts einer wässrigen Lösung in die Proben der mit der ersten Säure ausgerüsteten Filtermedium eingebracht wurden. Hierbei wurde der ph-Wert von wässrigen Lösung über meherere Eluationsstufen bestimmt:

Zunächst wurde ein Trägervlies aus Polyester-Spinnvliesstoff (Flächengewicht 125 g/m$^2$) ausschließlich mit Citronensäure und des weiteren mit einem Gemisch aus Citronensäure und Laurinsäure allergen-deaktivierend ausgerüstet. Die allergen-deaktivierende Dotierung erfolgte hierbei durch Aufbringen einer Citronensäurelösung bzw.einer Mischung der Wirkstoffe Citronensäure und Laurinsäure in wässriger Lösung auf das Filtermedium und nachfolgender Trocknung des nunmehr ausgerüsteten Vliesstoffs. Im Ergebnis erhielt man als Material 1 ein mit 10 Gew.% Citronensäure ausgerüstetes Filtermedium und als Material 2 ein mit 10 Gew.% Citronensäure und mit 5 Gew.% Laurinsäure ausgerüstetes Filtermedium.

Die Fragestellung war, ob Laurinsäure die Freisetzung von Zitronensäure verlangsamen kann und diese somit länger wirksam wäre. Dazu wurden 30 cm$^2$ große Proben der beiden unterschiedlich ausgerüsteten Filtermedien für eine Zeitspanne von 10 Sekunden unter Rühren mit einem Magnetrührer in ein Glasvial mit 15 ml Reinstwasser gegeben, danach aus dem Vial genommen und erneut für die gleiche Zeitspanne von 10 Sekunden in ein weiteres, neues Glasvial mit der gleichen Volumen von 15 ml Reinstwasser gegeben. Dieser Ansatz wurde mehrmals wiederholt. Im Anschluss wurde mittels pH-Meter der pH-Wert jeder Lösung bestimmt, um einen Unterschied zwischen Material 1 und Material 2 in deren zeitlichen Freisetzung von Citronensäure festzustellen. Dazu wurden jeweils in 3fach Bestimmung 30 cm$^2$ der o.g. Filtermedien mittels pH-Meter Modell Lab 860 der Fa. SI Analytics GmbH (55122 Mainz, Deutschland) geprüft. Vor den Messungen wurde das pH-Meter (Messbereich: pH: 2,000 - 19,999 (Genauigkeit +/- 0,005)) mit 2 definierten Pufferlösungen (pH= 4 und pH= 7, 2) mittels Punktkalibrierung kalibriert. Der pH- Wert wird mittels einer pH-Elektrode Modell SI Analytics 285129147 BlueLine 14 pH Electrode der Fa. SI Analytics GmbH (55122 Mainz, Deutschland) erfasst. Aufgrund der Temperaturabhängigkeit der pH-Wert-Bestimmung ist in o.g. pH-Elektrode einen Temperaturfühler integriert. Die Messungen erfolgten bei 25°C. Das der Bestimmung des pH-Werts über Elektroden zugrundeliegende Messprinzip ist die Potentiometrie: Eine mit Pufferlösung gefüllte Glasmembrankugel wird in die zu messende Flüssigkeit eingetaucht. Durch die Neigung der Wasserstoffionen, sich in dünner Schicht an Silikatgruppen der Glasoberfläche anzulagern, baut sich je nach pH-Differenz eine galvanische Spannung zwischen der Innen- und der Außenseite der Kugel auf. Diese elektromotorische Kraft wird mittels zweier Bezugselektroden gemessen, von denen sich eine innerhalb der Glaskugel, die andere in einem Referenzelektrolyten befindet. Im Falle der oben beschriebenen pH-Elektrode wird ein Silber/Silberchlorid (Ag/AgCl)- Referenzsystem verwendet:

**Tab.7**

| pH-Wert | Material 1 | | | Material 2 | | |
|---|---|---|---|---|---|---|
| **Prüfung** | 1. Prüfung | 2. Prüfung | 3. Prüfung | 1. Prüfung | 2. Prüfung | 3. Prüfung |
| **1. Glasviral** | 3,9 | 3,9 | 3,9 | 4,1 | 4,0 | 4,0 |
| **2. Glasviral** | 6,4 | 6,7 | 6,8 | 4,6 | 4,5 | 4,9 |
| **3. Glasviral** | 7,0 | 7,0 | 7,0 | 5,8 | 5,9 | 6,0 |
| **4. Glasviral** | | | | 6,5 | 6,7 | 7,0 |
| **5. Glasviral** | | | | 7,0 | 7,0 | 7,0 |

Im Ergebnis zeigt sich, dass das erfindungsgemäße Filtermedium nur eine geringe Verringerung der Verfügbarkeit auch bei - für Gasfiltermedien - extremen Bedingungen, d.h. beim mehrfachen Eintauchen in Wasser für eine Zeitspanne von 10 Sekunden erfährt.

**Patentansprüche**

1.   Filtermedium, insbesondere Medium zur Filtration von Gas-Partikelsystemen, umfassend mindestens eine säurefunktionalisierte Lage, die eine Fruchtsäure mit einem pks-1-Wert von 0 bis 7 als erste Säure aufweist, **dadurch gekennzeichnet, dass** die säurefunktionalisierte Lage eine C8- bis C18- Fettsäure als zweite Säure aufweist.

2.   Filtermedium nach Anspruch 1, **dadurch gekennzeichnet, dass** die erste Säure ausgewählt ist aus der Gruppe bestehend aus Äpfelsäure, Fumarsäure, Gluconsäure, Glycolsäure, Mandelsäure, Milchsäure, Oxalsäure, Salicylsäure, α-Hydroxycaprylsäure, Weinsäure, Citronensäure und Gemischen hiervon.

3. Filtermedium nach Anspruch 1 oder 2, **dadurch gekennzeichnet, dass** die zweite Säure ausgewählt ist aus der Gruppe bestehend aus C8- bis C16-Fettsäuren, C12- bis C14-Fettsäuren, C8-, C10-, C12-Fettsäuren und Gemischen hiervon.

4. Filtermedium nach einem oder mehreren der vorangehenden Ansprüche, **dadurch gekennzeichnet, dass** die zweite Säure Caprylsäure, Pelargonsäure, Caprinsäure, Undecansäure, Laurinsäure, Tridecansäure, Myristinsäure, Pentadecansäure, Palmitinsäure und/oder ein Gemisch hiervon ist.

5. Filtermedium nach einem oder mehreren der vorangehenden Ansprüche, **dadurch gekennzeichnet, dass** das Verhältnis zwischen erster Säure und zweiter Säure im Filtermedium von 10.000 :1 bis 1 : 1, vorzugsweise von 100 : 1 bis 5: 1 beträgt.

6. Filtermedium nach einem oder mehreren der vorangehenden Ansprüche, **dadurch gekennzeichnet, dass** die säurefunktionalisierte Lage die erste Säure in einer Menge von 0,1 Gew.% bis 30 Gew.%, enthält und/oder dass die säurefunktionalisierte Lage die zweite Säure in einer Menge von weniger als 10 Gew.%, jeweils bezogen auf das Geamtgewicht der säurefunktionalisierten Lage enthält.

7. Filtermedium nach einem oder mehreren der vorangehenden Ansprüche, **dadurch gekennzeichnet, dass** die säurefunktionalisierte Lage als imprägnierter und/oder beschichteter Vliesstoff ausgestaltet ist.

8. Filtermedium nach einem oder mehreren der vorangehenden Ansprüche, **dadurch gekennzeichnet, dass** bei der Herstellung der säurefunktionalisierten Lage die mit Säure zu funktionalisierende Lage vor und/oder gleichzeitig mit dem Aufbringen der zweiten Säure mit einer grenzflächenaktiven Substanz als Netzmittel behandelt wird.

9. Filtermedium nach einem oder mehreren der vorangehenden Ansprüche, **dadurch gekennzeichnet, dass** bei seiner Herstellung die mit Säure zu funktionalisierende Lage vor und/oder gleichzeitig mit dem Aufbringen der Fettsäure mit einer fungiziden Substanz behandelt wird.

10. Verwendung des Filtermediums nach einem oder mehreren der vorangehenden Ansprüche zur Herstellung von Gebäudezuluft-, Autoinnenraumzuluft- und/oder Raumluftfiltern.

11. Filteranordnung, insbesondere zur Filtration von Gas-Partikelsystemen, umfassend

A) einen partikelfilternden Bereich (1), umfassend

- eine Partikelfilterträgerlage (1a), sowie
- eine auf der Partikelfilterträgerlage (1a) angeordnete Mikrofaserlage (1b) und/oder Membranfilterlage (1b'),
- gegebenenfalls eine auf der der Partikelfilterträgerlage (1a) abgewandten Seite der Mikrofaserlage (1b) und/oder Membranfilterlage (1b') angeordnete Decklage (1c); und/oder

B) einen absorbierenden Bereich (2), umfassend

- eine Adsorptionsschicht (2a), sowie
- eine auf der Adsorptionsschicht (2a) angeordnete Adsorptionsträgerlage (2b),

**dadurch gekennzeichnet, dass** mindestens eine Lage ausgewählt aus Partikelfilterträgerlage (1a), Mikrofaserlage (1b), Membranfilterlage (1b'), Decklage (1c), Adsorptionsschicht (2a) und Adsorptionsträgerlage (2b) aus einem Filtermedium nach einem oder mehreren der Ansprüche 1 bis 9 gebildet ist.

12. Filteranordnung nach Anspruch 11, **dadurch gekennzeichnet, dass** die Adsorptionsschicht (1b) ein Adsorptionsmittel, ausgewählt aus der Gruppe bestehend aus Aktivkohlepartikeln, Ionentauschern und Zeolithen, umfasst.

13. Filteranordnung nach Anspruch 11 oder 12, **dadurch gekennzeichnet, dass** die Adsorptionsträgerlage (2b) und/oder die Partikelfilterträgerlage (1a) einen Vliesstoff, ausgewählt aus Spinnvliesstoffen, mit einem mittleren Faserdurchmesser im Bereich von 20 bis 60 μm und/oder Stapelfaservliesstoffen mit einem mittleren Faserdurch-

messer von 10 - 50 $\mu$m und/oder die Mikrofaserlage (1b) und/oder Membranfilterlage (1b') einen Vliesstoff, vorzugsweise ausgewählt aus Mikrofaservliesstoffen mit einem mittleren Faserdurchmesser von 1 $\mu$m bis 10 um, und/oder die Decklage (1c) einen Vliesstoff, vorzugsweise ausgewählt aus Spinnvliesstoffen, mit einem mittleren Faserdurchmesser im Bereich von 20 bis 60 $\mu$m und/oder Stapelfaservliesstoffen mit einem mittleren Faserdurchmesser von 10 - 50 $\mu$m umfasst.

**Claims**

1. Filter medium, in particular medium for the filtration of gas-particle systems, comprising at least one acid-functionalized layer, which comprises a fruit acid with pks1 value from 0 to 7 as first acid, **characterized in that** the acid-functionalized layer comprises a C8 to C18 fatty acid as second acid.

2. Filter medium according to Claim 1, **characterized in that** the first acid is selected from the group consisting of malic acid, fumaric acid, gluconic acid, glycolic acid, mandelic acid, lactic acid, oxalic acid, salicylic acid, $\alpha$-hydroxycaprylic acid, tartaric acid, citric acid and mixtures thereof.

3. Filter medium according to Claim 1 or 2, **characterized in that** the second acid is selected from the group consisting of C8 to C16 fatty acids, C12 to C14 fatty acids, C8, C10 and C12 fatty acids and mixtures thereof.

4. Filter medium according to one or more of the preceding claims, **characterized in that** the second acid is caprylic acid, pelargonic acid, capric acid, undecanoic acid, lauric acid, tridecanoic acid, myristic acid, pentadecanoic acid, palmitic acid and/or a mixture thereof.

5. Filter medium according to one or more of the preceding claims, **characterized in that** the ratio between first acid and second acid in the filter medium is from 10 000:1 to 1:1, preferably from 100:1 to 5:1.

6. Filter medium according to one or more of the preceding claims, **characterized in that** the acid-functionalized layer comprises a quantity of from 0.1% by weight to 30% by weight of the first acid and/or that the acid-functionalized layer comprises a quantity of less than 10% by weight of the second acid, in each case based on the total weight of the acid-functionalized layer.

7. Filter medium according to one or more of the preceding claims, **characterized in that** the acid-functionalized layer takes the form of impregnated and/or coated nonwoven fabric.

8. Filter medium according to one or more of the preceding claims, **characterized in that** during the production of the acid-functionalized layer the layer to be functionalized with acid is treated with a surfactant substance as wetting agent before and/or at the same time as the application of the second acid.

9. Filter medium according to one or more of the preceding claims, **characterized in that** during production thereof the layer to be functionalized with acid is treated with a fungicidal substance before and/or at the same time as the application of the fatty acid.

10. Use of the filter medium according to one or more of the preceding claims for the production of air-intake filters in buildings, air-intake filters for automobile interiors, and/or room-air filters.

11. Filter arrangement, in particular for the filtration of gas-particle systems, comprising

    A) a particle-filtering region (1) comprising

      - a particle filter support layer (1a), and also
      - arranged on the particle filter support layer (1a), a microfibre layer (1b) and/or membrane filter layer (1b'),
      - optionally an overlayer (1c) arranged on that side of the microfibre layer (1b) and/or membrane filter layer (1b') that faces away from the particle filter support layer (1a); and/or

    B) an absorbent region (2) comprising

      - an adsorption layer (2a), and also

- arranged on the adsorption layer (2a), an adsorption support layer (2b),

**characterized in that** at least one layer selected from particle filter support layer (1a), microfibre layer (1b), membrane filter layer (1b'), overlayer (1c), adsorption layer (2a) and adsorption support layer (2b) is composed of a filter medium according to one or more of Claims 1 to 9.

**12.** Filter arrangement according to Claim 11, **characterized in that** the adsorption layer (1b) comprises an adsorption means selected from the group consisting of activated charcoal particles, ion exchanges and zeolites.

**13.** Filter arrangement according to Claim 11 or 12, **characterized in that** the adsorption support layer (2b) and/or the particle filter support layer (1a) comprises a nonwoven fabric selected from spunbond nonwovens, with average fibre diameter in the range from 20 to 60 $\mu$m and/or staple fibre nonwoven fabrics with average fibre diameter of from 10 to 50 $\mu$m and/or the microfibre layer (1b) and/or membrane filter layer (1b') comprise a nonwoven fabric preferably selected from microfibre nonwovens with average fibre diameter from 1 $\mu$m to 10 $\mu$m, and/or the overlayer (1c) comprises a nonwoven fabric preferably selected from spunbond nonwovens, with average fibre diameter in the range from 20 to 60 $\mu$m and/or staple fibre nonwoven fabrics with average fibre diameter from 10 to 50 $\mu$m.

**Revendications**

**1.** Matériau filtrant, notamment matériau pour la filtration de systèmes gaz-particules, comprenant au moins une couche à fonctionnalisation acide, qui comprend un acide de fruit ayant une valeur de pks 1 de 0 à 7 en tant que premier acide, **caractérisé en ce que** la couche à fonctionnalisation acide comprend un acide gras en C8 à C18 en tant que deuxième acide.

**2.** Matériau filtrant selon la revendication 1, **caractérisé en ce que** le premier acide est choisi dans le groupe constitué par l'acide malique, l'acide fumarique, l'acide gluconique, l'acide glycolique, l'acide mandélique, l'acide lactique, l'acide oxalique, l'acide salicylique, l'acide $\alpha$-hydroxycaprylique, l'acide tartrique, l'acide citrique et leurs mélanges.

**3.** Matériau filtrant selon la revendication 1 ou 2, **caractérisé en ce que** le deuxième acide est choisi dans le groupe constitué par les acides gras en C8 à C16, les acides gras en C12 à C14, les acides gras en C8, C10, C12 et leurs mélanges.

**4.** Matériau filtrant selon une ou plusieurs des revendications précédentes, **caractérisé en ce que** le deuxième acide est l'acide caprylique, l'acide pélargonique, l'acide caprique, l'acide undécanoïque, l'acide laurique, l'acide tridécanoïque, l'acide myristique, l'acide pentadécanoïque, l'acide palmitique et/ou un mélange de ceux-ci.

**5.** Matériau filtrant selon une ou plusieurs des revendications précédentes, **caractérisé en ce que** le rapport entre le premier acide et le deuxième acide dans le matériau filtrant est de 10 000:1 à 1:1, de préférence de 100:1 à 5:1.

**6.** Matériau filtrant selon une ou plusieurs des revendications précédentes, **caractérisé en ce que** la couche à fonctionnalisation acide contient le premier acide en une quantité de 0,1 % en poids à 30 % en poids et/ou **en ce que** la couche à fonctionnalisation acide contient le deuxième acide en une quantité de moins de 10 % en poids, à chaque fois par rapport au poids total de la couche à fonctionnalisation acide.

**7.** Matériau filtrant selon une ou plusieurs des revendications précédentes, **caractérisé en ce que** la couche à fonctionnalisation acide est configurée sous la forme d'un non-tissé imprégné et/ou revêtu.

**8.** Matériau filtrant selon une ou plusieurs des revendications précédentes, **caractérisé en ce que**, lors de la fabrication de la couche à fonctionnalisation acide, la couche à fonctionnaliser avec des acides est traitée avec une substance tensioactive en tant qu'agent mouillant avant et/ou simultanément à l'application du deuxième acide.

**9.** Matériau filtrant selon une ou plusieurs des revendications précédentes, **caractérisé en ce que**, lors de sa fabrication, la couche à fonctionnaliser avec des acides est traitée avec une substance fongicide avant et/ou simultanément à l'application de l'acide gras.

**10.** Utilisation du matériau filtrant selon une ou plusieurs des revendications précédentes pour la fabrication de filtres de l'air d'alimentation de bâtiments, de l'air d'alimentation de l'espace intérieur d'automobiles et/ou de l'air ambiant.

**11.** Agencement de filtre, notamment pour la filtration de systèmes gaz-particules, comprenant :

A) une zone filtrant les particules (1), comprenant

- une couche support de filtre à particules (1a), et
- une couche de microfibres (1b) et/ou une couche de filtre à membrane (1b') agencées sur la couche support de filtre à particules (1a),
- éventuellement une couche de recouvrement (1c) agencée sur le côté opposé à la couche de support de filtre à particules (1a) de la couche de microfibres (1b) et/ou de la couche de filtre à membrane (1b') ; et/ou

B) une zone absorbante (2), comprenant

- une couche d'adsorption (2a) et
- une couche support d'adsorption (2b) agencée sur la couche d'adsorption (2a),

**caractérisé en ce qu'**au moins une couche choisie parmi la couche support de filtre à particules (1a), la couche de microfibres (1b), la couche de filtre à membrane (1b'), la couche de recouvrement (1c), la couche d'adsorption (2a) et la couche support d'adsorption (2b) est formée à partir d'un matériau filtrant selon une ou plusieurs des revendications 1 à 9.

**12.** Agencement de filtration selon la revendication 11, **caractérisé en ce que** la couche d'adsorption (1b) comprend un agent d'adsorption, choisi dans le groupe constitué par les particules de charbon actif, les échangeurs d'ions et les zéolithes.

**13.** Agencement de filtration selon la revendication 11 ou 12, **caractérisé en ce que** la couche support d'adsorption (2b) et/ou la couche support de filtre à particules (1a) comprend un non-tissé, choisi parmi les non-tissés de filaments, ayant un diamètre de fibre moyen dans la plage allant de 20 à 60 $\mu$m et/ou les non-tissés de fibres discontinues ayant un diamètre de fibre moyen de 10 à 50 $\mu$m et/ou la couche de microfibres (1b) et/ou la couche de filtre à membrane (1b') comprennent un non-tissé, de préférence choisi parmi les non-tissés de microfibres ayant un diamètre de fibre moyen de 1 $\mu$m à 10 $\mu$m, et/ou la couche de recouvrement (1c) comprend un non-tissé, de préférence choisi parmi les non-tissés de filaments, ayant un diamètre de fibre moyen dans la plage allant de 20 à 60 $\mu$m, et/ou les non-tissés de fibres discontinues ayant un diamètre de fibre moyen de 10 à 50 $\mu$m.

Fig.1

Fig.2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2879776 A1
- US 20130183879 A1

**In der Beschreibung aufgeführte Nicht-Patentliteratur**

- **DANIEL C. HARRIS.** Lehrbuch der quantitativen Analyse. Springer Verlag, 2014